# EUROPEAN PATENT APPLICATION

(11) **EP 2 073 009 A1**
(43) Date of publication of application: **24.06.2009**
(21) Application number: 07450236.0
(22) Date of filing: 19.12.2007
(51) Int. Cl.: G01N 33/569

(54) **Method for T, NK and NKT cells**

(71) Applicant: Cell Med Research GMBH, 3502 Krems-Lerchenfeld (AT)
(72) Inventor: Rubiolo, Cristina, 1220 Vienna (AT)
(74) Representative: Heger, Georg

(57) **Abstract**

The present invention relates to a method for determining specifically and for discriminating T cells, Natural Killer (NK) cells and Natural Killer T (NKT) cells in a sample by incubating said sample with at least eight antibodies binding to at least eight different T cell surface markers, NK cell surface markers and NKT cell surface markers selected from the group consisting of CD3, TCR α/β, TCR γ/δ, CD4, CD8, CD45RO, CD25, CD28, CCR4, CD94, CD38, CD62L, CCR7, CD56, CD69, CD158a, Nkp44, HLA-DR and CD161.

## Description

The present invention relates to a method for determining specifically and for discriminating T cells, Natural Killer (NK) cells and Natural Killer T (NKT) cells in a blood sample.

For instance, the development of effective tumor vaccines, such those based on the reiterated inoculation of antigen loaded dendritic cells,requires an accurate monitoring of the immune response in order to improve both the preparation and the route of administration and therefore increasing the clinical relevance of such a therapy. One major aim of the immune monitoring is to determine the efficacy of a vaccine to induce or increase a specific T-cell and NKs response. Further objectives relate to the prevalence and functional relevance of spontaneous tumor-directed immune responses, the functional characteristics of T-cell and NK responses, and the relationship between immune monitoring assay results and clinical end points. The issue of T-cell and NKs monitoring has become more complex as different types and generations of assays have been adopted during the past decade and both standardization and validation of assays have often been insufficient. Suitable animal models for immune monitoring are, however, lacking, because preclinical vaccine development in rodents does not allow serial immune monitoring of the peripheral blood, as it is commonly used in patients. Presently, either in routine clinical practice or in research setting, treatment efficacy is always assessed by serial history and physical examinations, radiographic studies, as well as evaluation of serologic tumor markers (Ellis et al. Histopathology. (1999) 34:372). Nevertheless, each of these approaches results inaccurate. Therefore, simpler, faster and above all more effective means of assessing therapy effectiveness are required, in order, on the one hand, to optimize individual treatment for each patient and, on the other hand, to serve as surrogate measures of effectiveness during the establishment of new therapies. The current situation is as well characterized by a lack of universal standards for T-cell assessment, uncertainty about the association between immune monitoring assay results and clinical anti-tumor end points, and lack of knowledge regarding the contribution of different aspects of T-cell and NKs function to clinical efficacy (Keilholz et al. (2006). Clin Cancer Res. 12: 2346s-2353s).

It is an object of the present invention to provide suitable methods and means, which allow to determine the immune status of an individual or a patient who is treated with different therapies, such as but not exclusively immune therapy, chemotherapy and radiotherapy.

Therefore the present invention relates to a method for determining specifically and for discriminating T cells, Natural Killer (NK) cells and Natural Killer T (NKT) cells in a sample comprising the steps of:
a) providing a sample comprising leukocytes, in particular T cells, NK cells and NKT cells, or parts thereof,
b) incubating said sample with at least eight antibodies binding to at least eight different T cell surface markers, NK cell surface markers and NKT cell surface markers, wherein the T cell surface marker is selected from the group consisting of CD3, TCR α/β, TCR γ/δ, CD4, CD8, CD45RO, CD25, CD28, CCR4, CD94, CD38, CD62L, CCR7 and CCR7, the NK cell surface marker is selected from the group consisting of CD56, CD28, CD69, CCR4, CD158a, Nkp44, CD94, CD38, CD62L, CD8, HLA-DR, CD25 and CD161, and the NKT cell surface marker is selected from the group consisting of CD56, CD3, CD8, CCR4 and CD69, wherein the antibodies used for the incubation bind to at least one T cell surface marker, at least one NK cell surface marker and at least one NKT cell surface marker,
c) detecting the binding of at least one of said antibodies to said cells or parts thereof, and
d) determining T cells, NK cells and/or NKT cells in the sample when at least one of said antibodies binds to said cells or parts thereof and/or determining T cells in the sample when the binding of antibodies directed to CD3, TCR α/β, TCR γ/δ, CD4, CD8, CD45RO, CD25, CD28, CCR4, CD94, CD38, CD62L, FoxP3 and CCR7 to said cells or parts thereof and no binding to a NK cell surface marker or NKT cell surface marker is detected, determining NK cells in the sample when the binding of antibodies directed to CD56, CD28, CD69, CCR4, CD158a, Nkp44, CD94, CD38, CD62L, CD8, HLA-DR, CD25 and CD161 to said cells or parts thereof and no binding to a T cell surface marker or NKT cell surface marker is detected and/or determining NKT cells in the sample when the binding of antibodies directed to CD56, CD3, CD8, CCR4 and CD69 to said cells or parts thereof and no binding to a T cell surface marker or NK cell surface marker is detected.

The method according to the present invention allows an efficient immune monitoring (IM), based on the detection of 19 different surface markers, in order to differentiate T-cells, NKs and NKTs and their status as active, inactive, de-activated, resting, immature, mature. Moreover, the method according to the present invention allows to differentiating cytotoxic T-cells from T-reg and T-mem. T-mem are further differentiated as central memory and intermediate memory. The cells are as well characterized as able to respond to stimuli triggered by dendritic cells, to produce interleukins and INF-γ. The data may be confirmed by ELISA assays and/or by co-culture with dendritic cells.

The method of the present invention is based on the phenotypical analysis of surface markers by using antibodies, which bind specifically to said markers.

Due to the information about the immune status of the patient that the method of the present invention provides, it is particularly suitable to monitoring the efficacy of cancer therapies, for example of an immune therapy, based on the inoculation of dendritic cells. The analysis allows in particular to estimate the number of T-reg, T-mem and T-cyt, to determine how many cells are active, how many cannot be activated by the therapy, how many can be deactivated by the therapy, etc.. In this way it is possible to determine if the therapy is successful (less T-reg than before starting the therapy, more T-cells activated and less deactivated or inactivated, etc.). At the same time, subsequent analyses allow to determine at which intervals the therapy is rather administrated and when it is better to perform clinical analyses to determine the efficacy of the therapy.

The method of the present invention can be performed quickly in any laboratory. It is particularly suitable to trials with new immune approach in cancer therapy or to trials, which require continous immune monitoring in order to determine the best route of administration, the best dosage and the best clinical points when performing clinical analyses. Such method is accurate but not invasive. It allows to determine both favorable and unfavorable effects of the tested medicament on the immune system of the patient (i.e. T-cells, NK and NKT) by showing the activation status of such component by analysing their phenotype along with the treatment. The analysis provide data about the number of cells belonging to each group (i.e. T-cells, NK and NKT)and sub-group (active, activated, inactive, inactivated, de-activated, immature, mature, etc...)

The method of the present invention allows to monitor the amount and the status of T-cells, NKTs and NKs in a sample of a patient, suffering from a disease, like solid or blood cancer, before, during and after the respective therapy, such as but not only, immune therapy, chemotherapy or radiotherapy. Comparison between the results obtained before starting, during and after the therapy may provide valuable information about the optimal dose of the medicament and the better route of administration in order to improve the therapy by individualization. Such a method may be particularly interesting to standardize the immune therapy, based on, e.g., reiterated inoculations of autologous, mature and loaded dendritic cells, especially if related to clinical parameters.

The sample used in the method of the present invention is incubated with at least eight antibodies binding to at least eight out of 19 T cell, NK cell and NKT cell markers disclosed above. However, it is of course possible to use at least nine, ten, eleven, twelve, 13, 14, 15, 16, 17, 18 or even 19 antibodies binding to the corresponding number of surface markers. Since some of surface markers are present on more than one type of cells (e.g. CD56 is a cell surface marker for NK and NKT cells, CD8 is a cell surface marker for T, NK and NKT cells, CD3 is a cell surface marker for T and NKT cells) a minimum of at least eight antibodies binding to at least eight different cell surface markers is required to identify and/or discriminate between T, NK and NKT cells. In order to increase the accuracy of the method it is of course possible to use more than eight antibodies. Table 1 summarized the function of the markers, which may be used to discriminate between the different cell types and their functionality grade.

**Table 1:**

| **Cell Type** | **Marker** | **Phenotype** |
|---|---|---|
| T-cells | CD3 | |
| | TCR α/β | |
| | TCR γ/δ | |
| | CD4 | Th |
| | CD8 | T-cytoxic |
| | CD8+ TCR α/β | T-cytoxic |
| | CD4+TCR α/β | Th |
| | CD45RO | Memory and active |
| | CD25 | Active |
| | CD45RO+CD25 | Active upon infection |
| | CD4+CD25 | T-reg |
| | CD45RO+CD4 | Resting |
| | CD45RO+CD8 | Resting |
| | CD28 | Active upon DCs contact |
| | CD8+CD28 | Active T-cytotoxic |
| | CCR4 | Activation of Th1 |
| | CD94 | Active |
| | CD38 | Active upon Tumor Progression |
| | CD62L | High production if IL-10 if highly expressed |
| | CD62L+CD38 | Active |
| | CD62L+CCR7 | Non-polarized/experienced T-mem |
| | CCR7 | Intermediate T-mem |
| NK cells | CD56 | |
| | CD28 | Active upon DCs contact |
| | CD69 | Active |
| | CD28 | Active upon DCs contact |
| | CCR4 | Inactive |
| | CD158a | Down-regulation of T-cell activity |
| | CCR4+CD158a | Down-regulation of T-cell activity |
| | Nkp44 | Tumor Cells Lysis |
| | CD94 | Migration and Cytotoxic Activity |
| | Nkp44+CD94 | Migration and Cytotoxic Activity |
| | CD38 | Migration and Cytotoxic Activity |
| | Nkp44+CD38 | Migration and Cytotoxic Activity |
| | CD62L | Secretion of cytokines |
| | CD62L+CD8 | Induction of DCs Activity |
| | HLA-DR | |
| | CD25 | Increased secretion of INF-γ |
| | CD25+HLA-DR | Increased secretion of INF-γ |
| | CD161 | Immature NKs |
| | CD161+HLA-DR | Immature NKs |
| NKT cells | CD56+CD3 | |
| | CD8+CCR4 | Supression of Tumor-specific T-cell activity |
| | CD8+CCR4 | Supression of Tumor-specific T-cell activity |
| | CD8+CD69 | Active |
| | CD8 | Active |

The incubation of the antibodies with the sample and the detection of the binding of said antibodies to the cell surface markers can be done simultaneously, i.e. the at least eight antibodies are contacted at the same time with the sample, or by grouping the antibodies.

The term "antibody", as used herein, is used in the broadest sense and specifically covers, for example, single monoclonal antibodies (including murine, chimerized or humanized antibodies), antibody compositions with polyepitopic specificity, single-chain antibodies, immunoconjugates and fragments of antibodies. In the method according to the present invention monoclonal antibodies are preferably used.

"Monoclonal antibodies" as used herein refer to antibodies obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations, which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. For example, the monoclonal antibodies to be used in accordance with the present invention may be made by the hybridoma (murine or human) method first described by Kohler et al. (Nature, 256:495 (1975)) or may be made by recombinant DNA methods.

"Antibody fragments", which may also be used in the method of the present invention, comprise a portion of an intact antibody, preferably comprising the antigen-binding or variable region thereof. Examples of antibody fragments include less than full length antibodies, Fab, Fab', F(ab')2, and Fv fragments, diabodies, linear antibodies, single-chain antibodies, single domain antibody molecules, fusion proteins, recombinant proteins and multispecific antibodies formed from antibody fragment(s).

An "intact" antibody is one, which comprises an antigen-binding variable region as well as a light chain constant domain (CL) and heavy chain constant domains, CH1, CH2 and CH3. The constant domains may be native sequence constant domains (e.g. human native sequence constant domains) or amino acid sequence variant thereof. Preferably, the intact antibody has one or more effector functions.

Whether an antibody according to the present invention binds to an antigen can be determined e.g. by Fluorescence-Activated Cell Sorter (FACS), enzyme-linked immunosorbent assay (ELISA) or an equivalent method.

According to a preferred embodiment of the present invention active T cells, NK cells and/or NKT cells present in the sample are determined by detecting the binding of at least one of said antibodies to said cells or parts thereof, wherein active T cells are determined when the binding of antibodies to CD45RO, CD25, CD28, CCR4, CD94, CD38 and/or CD62L to cells in the sample is detected, active NK cells are determined when the binding of antibodies to CD69, CD28, CD158a, Nkp44, CD94, CD38, CD25 and/or CD62L to cells in the sample is detected, and active NKT cells are determined when the binding of antibodies to CD8 and/or CD69 to cells in the sample is detected.

According to a further embodiment of the present invention inactive NK cells present in the sample are determined by detecting the binding of at least one of said antibodies to said cells or parts thereof, wherein inactive NK cells are determined when the binding of antibodies to CCR4 to cells in the sample is detected.

According to another embodiment of the present invention resting T cells present in the sample are determined by detecting the binding of at least one of said antibodies to said cells or parts thereof, wherein resting T cells are determined when the binding of antibodies to CD45RO is simultaneous to the binding to CD4 or CD8.

According to a preferred embodiment of the present invention immature NK cells present in the sample are determined by detecting the binding of at least one of said antibodies to said cells or parts thereof, wherein immature NK cells are determined when the binding of antibodies to CD161 and/or HLA-DR to cells in the sample is detected.

The method according to the present invention allows discrimination between T cell types. Therefore it is preferred that the T cells present in the sample are differentiated in cytotoxic T cells, regulatory T cells and memory T cells, wherein cytotoxic T cells are determined when the binding of antibodies to CD8 and/or TCRα/β to cells in the sample is detected, regulatory T cells are determined when the binding of antibodies to CD4 and/or CD25 to cells in the sample is detected and memory T cells are determined when the binding of antibodies to CCR7 to cells in the sample is detected.

The sample to be analyzed in the method according to the present invention comprises leukocytes or is suspected to comprise said cells. The sample is therefore preferably blood, preferably peripheral blood, lymphonodal fluid, tumor lysate, leukaphereses and elutriation. Of course it is also possible to analyse a sample from a cell culture, which may comprise leukocytes.

In order to facilitate the detection of the binding of the antibodies to the cells, the antibodies used in the method of the present invention are labelled with a marker (label). Said marker is preferably fluorescent or chemoluminescent. Of course it is also possible to use secondary antibodies, which are labelled and bind to the first antibodies bound on the cells. The label on the antibody may also be selected from the many known diagnostic labels, such as radioactive compounds, fluorescent compounds and proteins, calorimetric enzymes, etc. In one embodiment, the label can be a colorimetric enzyme, which upon contact with a substrate produces a detectable color signal.

The label preferably used in the method of the present invention is selected from the group consisting of fluorescein isothiocyanate (FITC), phycoerythrin (PE), allophycocyanin (APC), PE-cyanin-5 (PC5), PE-cyanin-7 (PC7), PE-cyanin-5.5, PE-Texas Red (ECD), rhodamine, PerCP and Alexa dyes.

According to a further embodiment of the present invention the detection of binding of the antibodies to the cells is performed by flow cytometry.

According to a preferred embodiment of the present invention the following markers are used during the immune monitoring:

| **FITC** | **PE** | **ECD** | **PC5** | **PC7** |
|---|---|---|---|---|
| TCR γ/δ | TCR α/β | CD3 | CD4 | CD8 |
| CD25 | CD45RO | CD3 | CD4 | CD8 |
| CD28 | CD69 | CD3 | CD56 | CD8 |
| CD158a | CCR4 | CD3 | CD56 | CD8 |
| CD94 | NKp44 | CD3 | CD56 | CD38 |
| CD62L | CCR7 | CD3 | CD56 | CD8 |
| CD25 | HLA-DR | CD3 | CD161 | CD56 |

Another aspect of the present invention relates to a kit for determining specifically and for discriminating T cells, Natural Killer (NK) cells and Natural Killer T (NKT) cells in a sample comprising antibodies directed to CD3, TCR α/β, TCR γ/δ, CD4, CD8, CD45RO, CD25, CD28, CCR4, CD94, CD38, CD62L, CCR7, CD56, CD69, CD158a, Nkp44, CD38, HLA-DR and CD161, wherein the antibodies are fluorescently labelled.

According to a preferred embodiment of the present invention the label is selected from the group consisting of fluorescein isothiocyanate (FITC), phycoerythrin (PE), allophycocyanin (APC), PE-cyanin-5 (PC5), PE-cyanin-7 (PC7), PE-cyanin-5.5, PE-Texas Red (ECD), rhodamine, PerCP and Alexa dyes.

Yet another aspect of the present invention relates to the use of a kit according to the present invention for determining specifically and for discriminating T cells, Natural Killer (NK) cells and Natural Killer T (NKT) cells in a sample.

The present invention is further illustrated by the following figures and examples, however, without being restricted thereto.

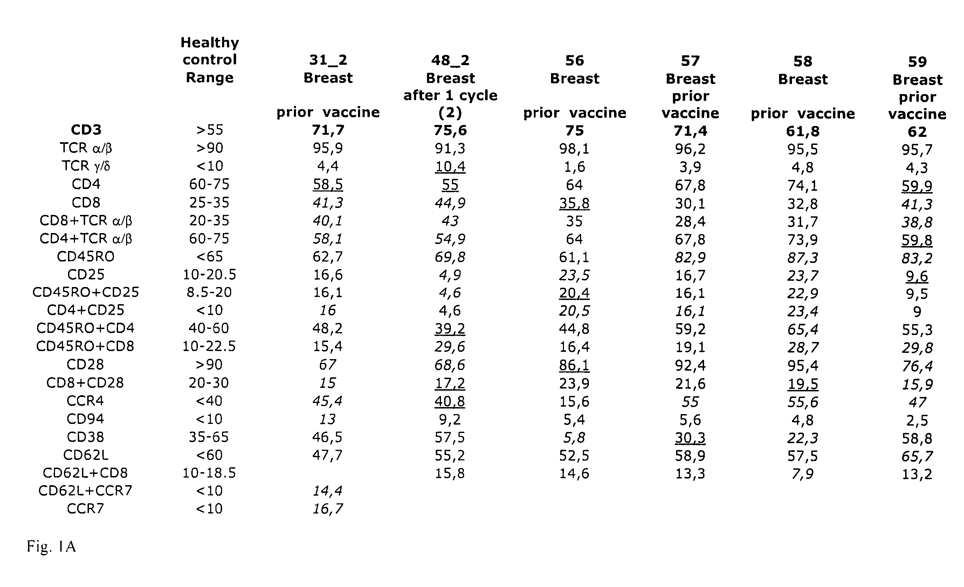

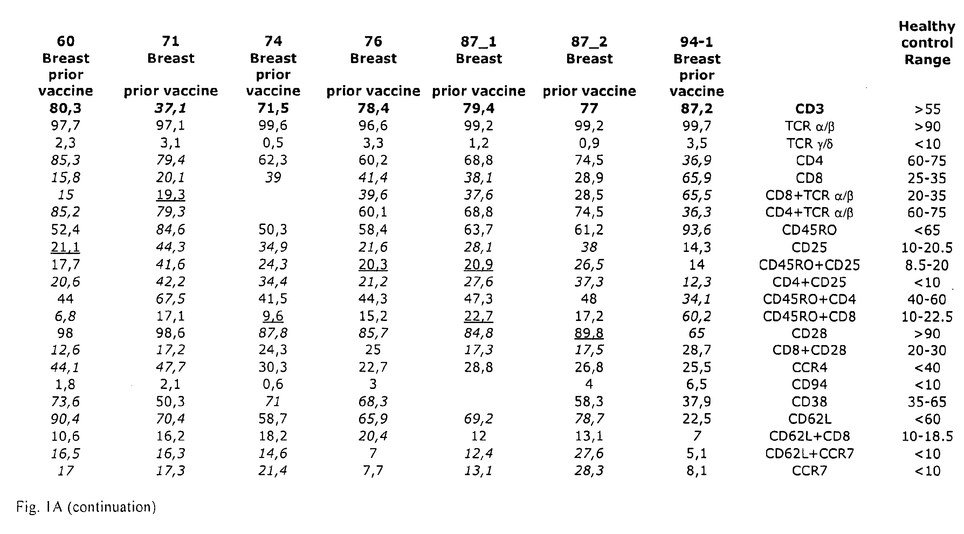

Fig. 1A shows T-cell quantification and characterization in patients suffering from breast cancer.

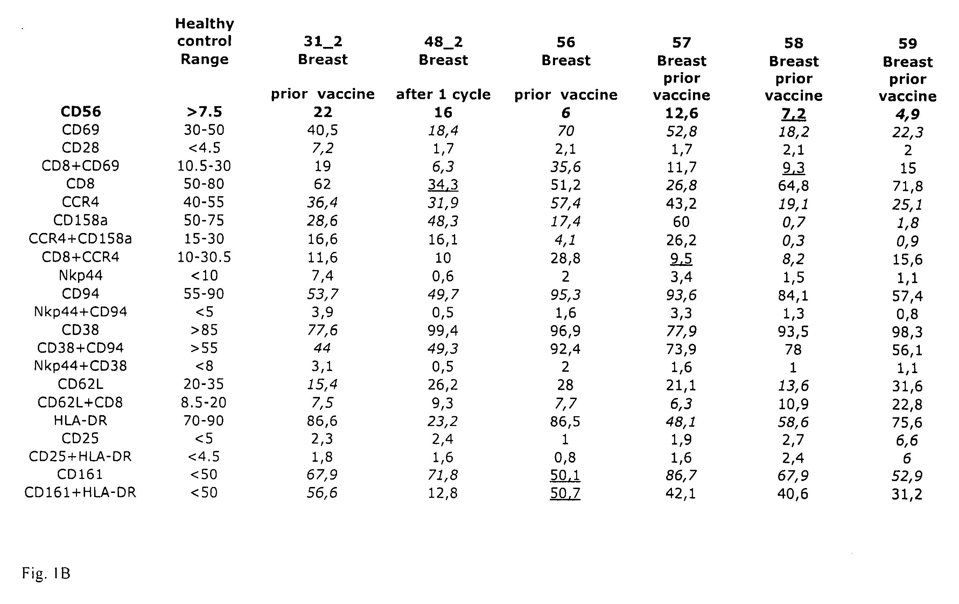

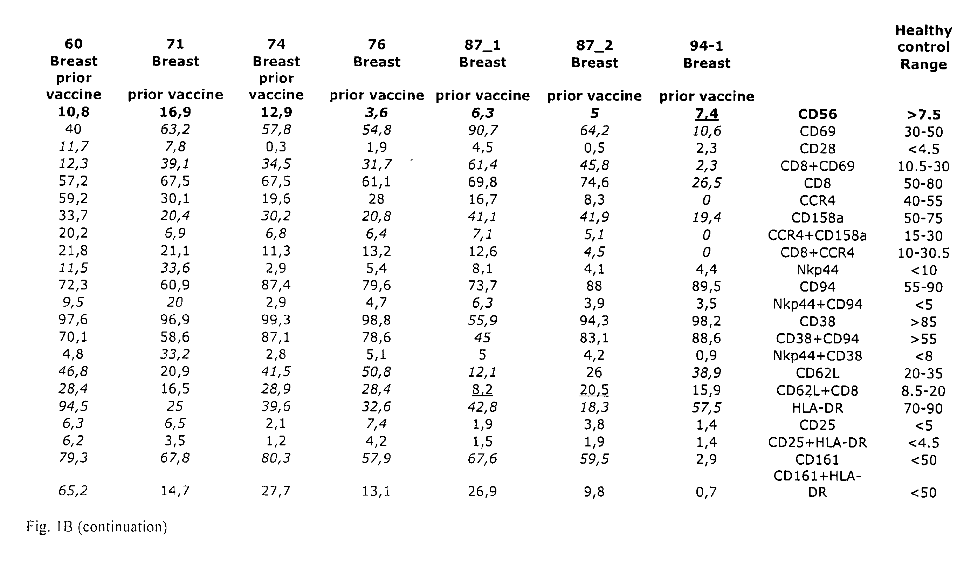

Fig. 1B shows NK and NKT quantification and characterization in patients suffering from breast cancer.

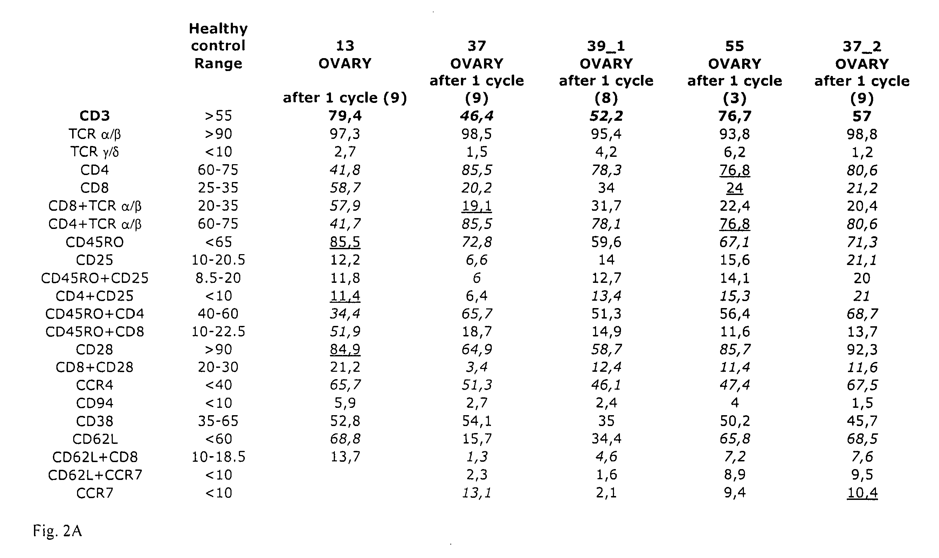

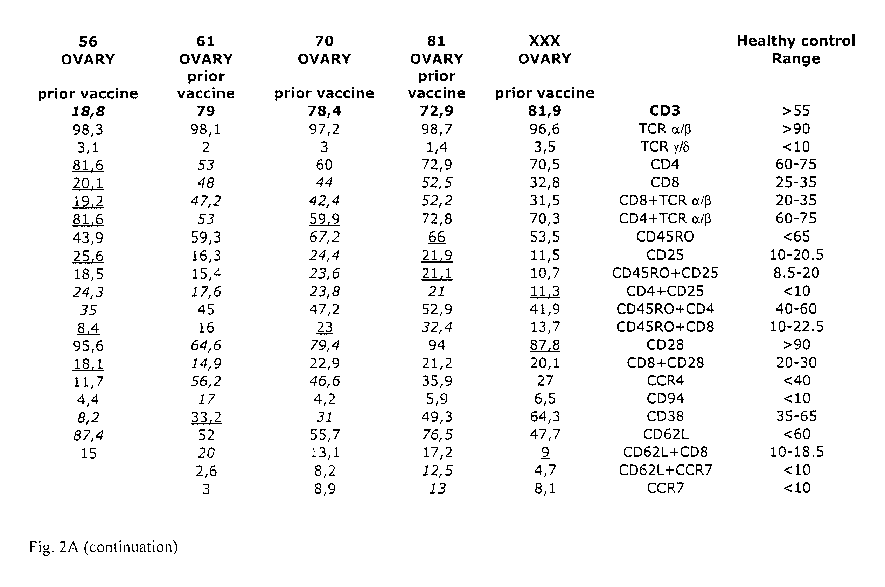

Fig. 2A shows T-cell quantification and characterization in patients suffering from ovarian cancer.

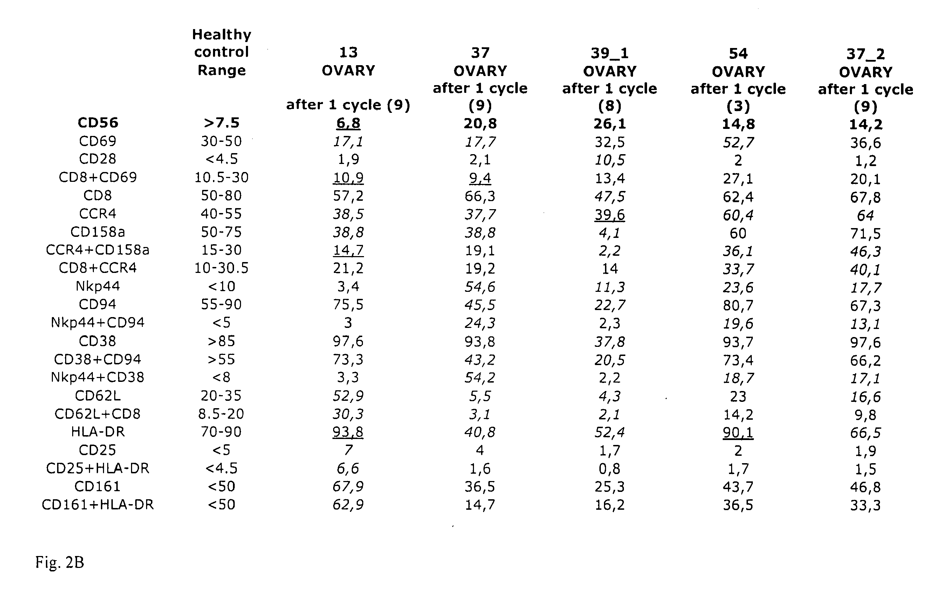

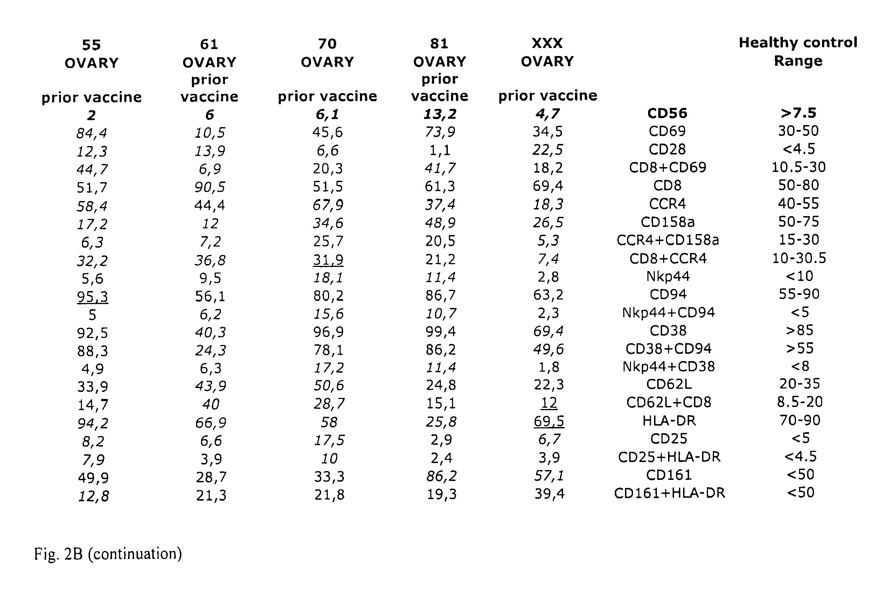

Fig. 2B shows NK and NKT quantification and characterization in patients suffering from ovarian cancer.

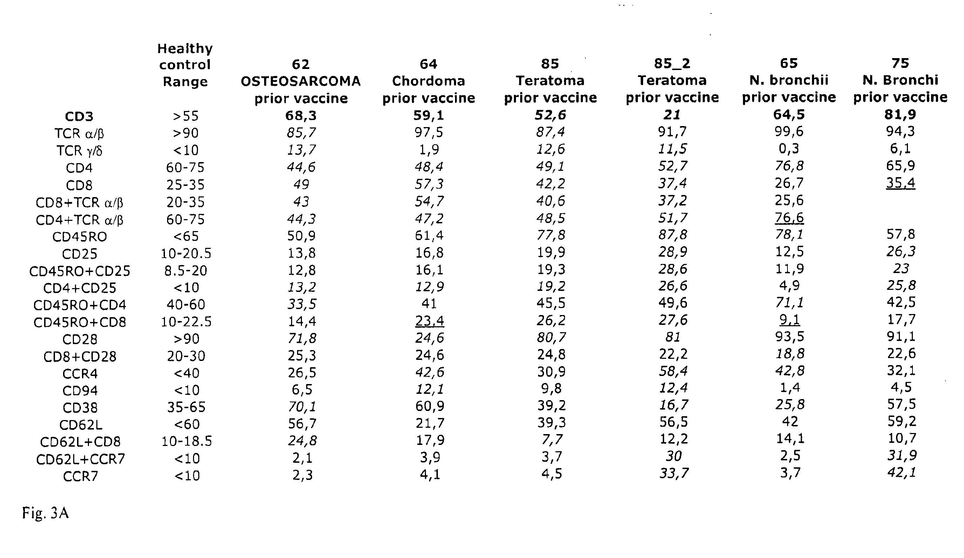

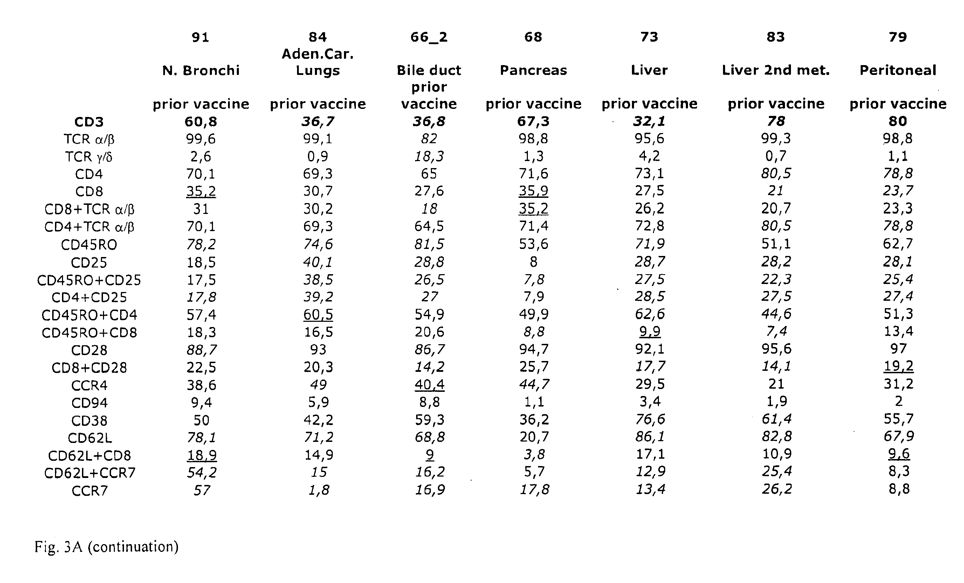

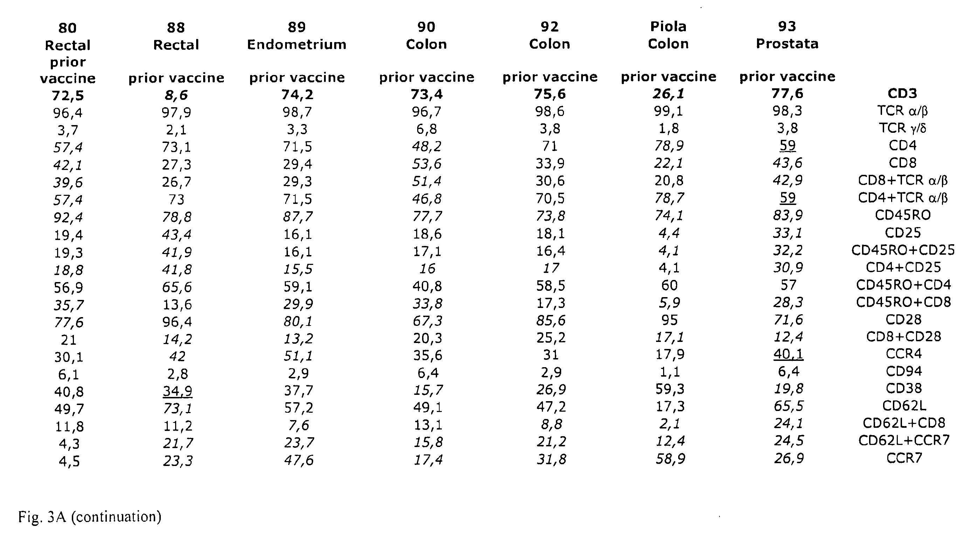

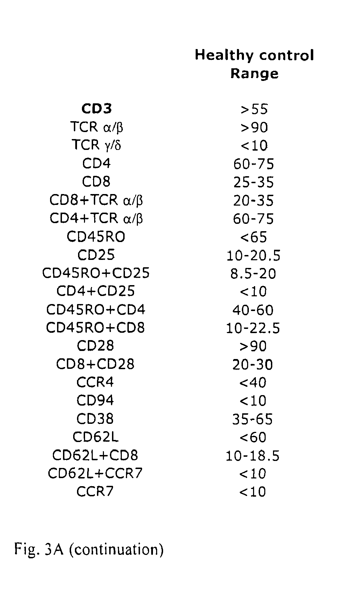

Fig. 3A shows T-cell quantification and characterization in patients suffering from solid cancer.

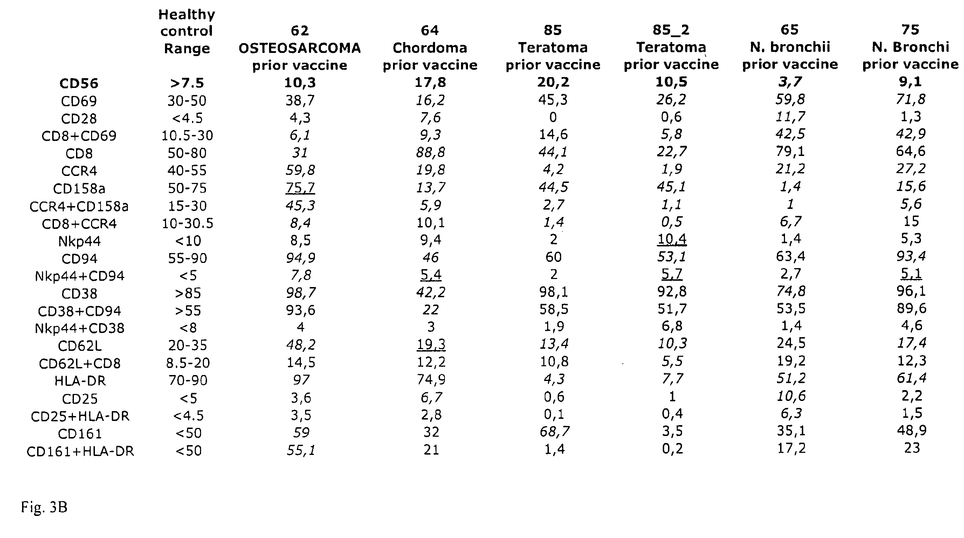

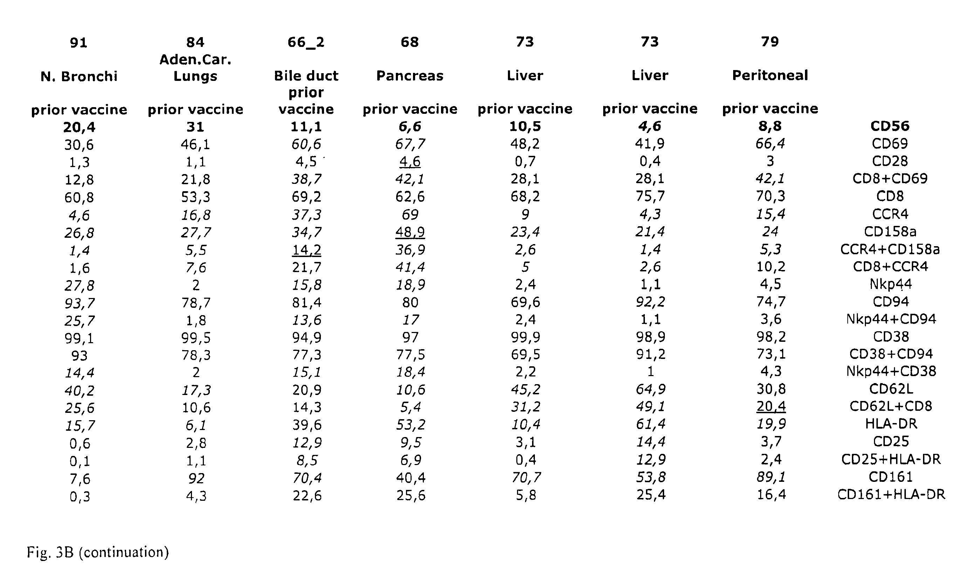

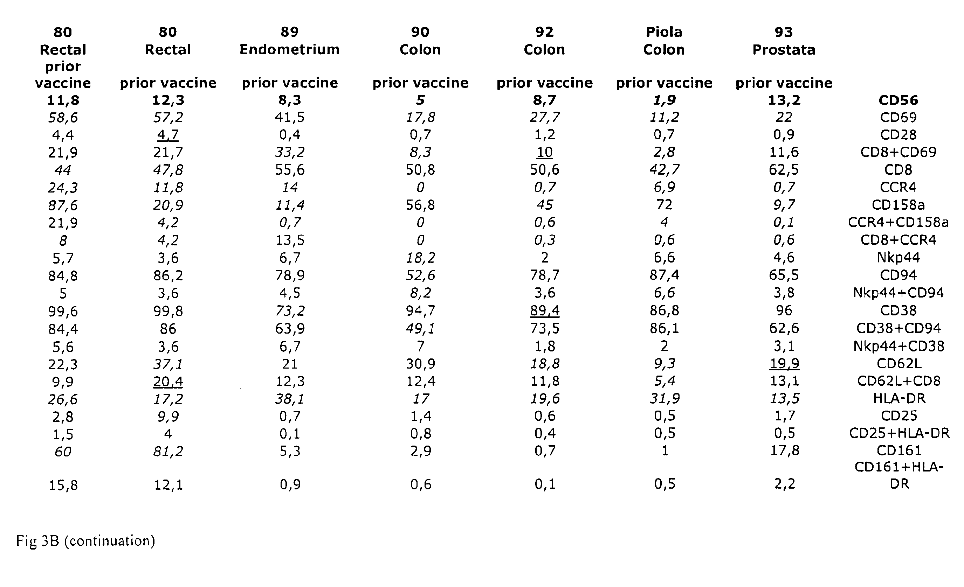

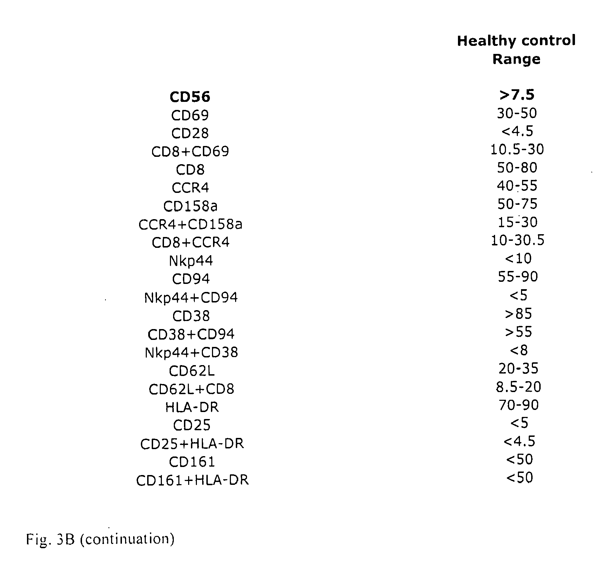

Fig. 3B shows NK and NKT quantification and characterization in patients suffering from solid cancers.
Fig. 4A: T-cell quantification and characterization in an ovarian cancer patient, treated with immune therapy.
Fig. 4B: NK and NKT quantification and characterization in an ovarian cancer patient, treated with immune therapy.

### EXAMPLE:

### Methods

1 mL of cancer patient's peripheral blood was collected in EDTA or Eparin tubes. The sample was immediately processed. Alternatively, the sample can be stored at 4°C for max 48h. In case the sample is not processed in loco, it must be transported at a temperature between 4-8°C (long-lasting transport, i.e. more than 4h) or at RT (quick transport, i.e. less than 4h).

The sample was divided in 8 aliquots of 50 µl each and incubated 20 min with the proper mix of FACS antibodies or of corresponding IgG. Thereafter each aliquot was washed in PBS thrice and processed by FACS.

The following markers are used during the immune monitoring:

| **FITC** | **PE** | **ECD** | **PC5** | **PC7** |
|---|---|---|---|---|
| TCR γ/δ | TCR α/β | CD3 | CD4 | CD8 |
| CD25 | CD45RO | CD3 | CD4 | CD8 |
| CD28 | CD69 | CD3 | CD56 | CD8 |
| CD158a | CCR4 | CD3 | CD56 | CD8 |
| CD94 | NKp44 | CD3 | CD56 | CD38 |
| CD62L | CCR7 | CD3 | CD56 | CD8 |
| CD25 | HLA-DR | CD3 | CD161 | CD56 |

### FACS (Fluorescent-activated cell sorting) analysis

50 µL of whole blood or 50 µL of cell-suspension (thawed DCs, in a final concentration of 0.1-1 x 10⁶ cells/mL in PBS, 1%BSA, 0.1% NaN₃) were incubated 15' at room temperature with the appropriate amount of FACS antibodies, according to the manufactures recommendations, washed at 800 rpm (RT) with 1.5 mL or 0.8 mL of PBS respectively, resuspended in 0.5 mL of PBS, (eventually supplemented with fixative) and further processed by FACS®

### ELISA

1 x 10⁶ peripheral blood cells/mL were cultured in RPMI supplemented with 2% HS at a final concentration of. After 24h, the supernatant was harvested and analysed for human IL-2, IL-4, IL-10 ad IL-12p70, using OptEIA ELISA Sets (BD Pharmingen) protocol: 6 wells were coated with IL-10 or IL-12 antibody and incubated overnight at 4°C. After blocking the wells, the samples and the standard dilutions were incubated for 2h at RT. Afterwards the wells were incubated in one step with biotinylated IL-2, IL-4, IL-10 or IL-12 antibody respectively, and with streptavidin horseradish peroxidase. After 30 min the substrate solution (TMB) is added and 30 min later the colour reaction is stopped with phosphoric or sulphuric acid. The absorbance, which was directly related to the amount of detected interleukin and corresponding to the standard dilutions, was measured at an absorbance of 450 nm with wavelength correction at 620 nm.

### Mixed leukocyte reaction

1 x 10⁵ peripheral blood cells/well were co-cultured with 1 x 10⁴ heterologous DCs/well in a 96-well U-bottom plate in RPMI with 2% human serum (off the clott, AB), supplemented with 10% AlamarBlue at a final volume of 150 µL. Proliferation causes reduction of the dye changing from oxidized (non-fluorescent, blue) form to reduced (fluorescent, red) form. For 2-7 days, every 4-12h, the absorbance was measured in a plate-reader at 595 nm with reference wavelength at 620 nm.

### Results

All the results showed in this section were performed starting by 1 mL sample of cancer patients' peripheral blood.

An immuno monitoring (IM) panel, which comprises 19 surface markers, in order to differentiate T-cells, NKs and NKTs and their status as active, inactive, de-activated, resting, immature, mature was used. Moreover, said markers were used to differentiate cytotoxic T-cells from T-reg and T-mem. T-mem were further differentiated as central memory and intermediate memory.

A "healthy control range" expression for the markers mentioned above, as result of the mean among the values obtained from 10 healthy controls, was determined.

The IM was tested in 10 patients with ovarian cancer, in 10 patients with breast cancer, in 1 patient with osteosarcoma, in 1 patient with chordoma, in 2 patients with N. bronchi, in 1 patient with pancreas cancer, in 2 patient with liver cancer, in 1 patient with peritoneal carcinoma and in 2 patients with rectal cancer.

In the group of patients with ovarian cancer, 5 patients were tested before undergoing the 1st cycle of immune therapy (9 injections of autologous, semi-mature, unloaded DCs) and 5 were tested 9 months after 1 cycle (3 patients with a cycle of 9 injections, 1 with a cycle of 3 injections and 1 with a cycle of 4 injections).

In the group of patients with breast cancer, 9 patients were tested before undergoing the 1st cycle of immune therapy and 1 was tested 4 months after 1 cycle of 2 injections.

In the group of patients with different cancers, all the patients were tested before undergoing the 1st cycle of immune therapy.

Patients with ovarian cancer show on average higher deviation from the standard number of CD3+ cells, in comparison with patients with breast cancer or with other tumor type under investigation.

Independently from cancer type, all tumor patients show deviation in the ratio between CD4+ and CD8+ cells in comparison to healthy control and higher levels of CD4+ CD25+ T-cells (T-reg) and lower expression of CD8+CD28+ T-cells (T-cytoxic). Moreover, the number of CD62L+ cells is increased (production of IL-10 and consequent immune supression).

On the other side, the analysis of the NKs and NKTs cells showed that breast cancer patients tend to have more immature NKs (CD161+) in comparison with healthy controls and ovarian cancer patients.

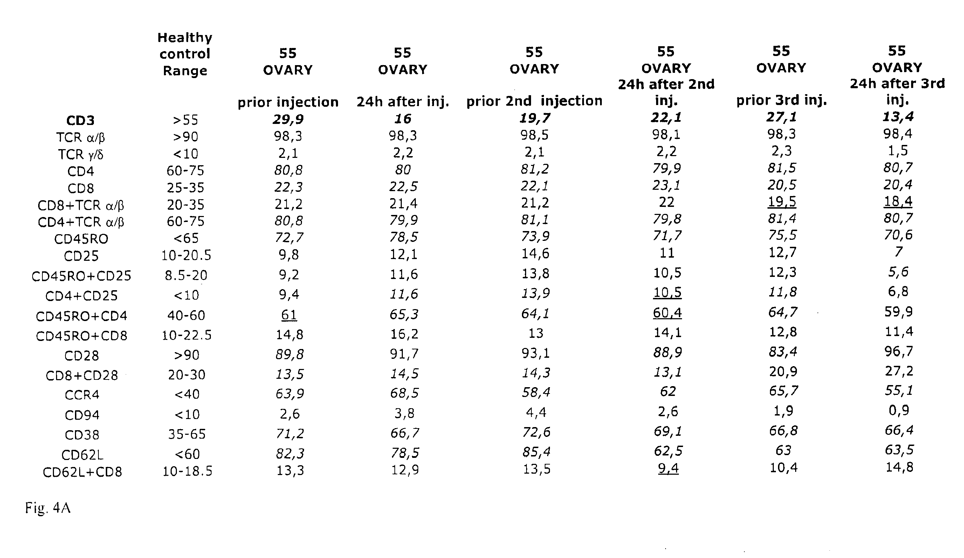

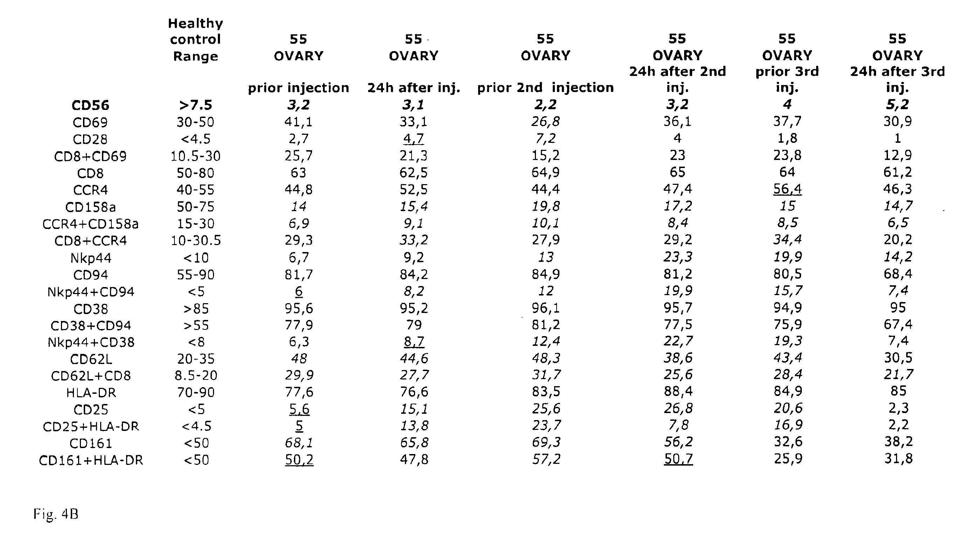

Data about the efficacy of the immune therapy (3 injections) on the basis of the IM results showed that the immune therapy did not increase the number of T-cells and only very slightly that of NKs without improving the ratio between CD4+ and CD8+ cells. In any case, it could decrease the number of CD4+ CD25+ T-cells and increase that of CD8+CD28+ T-cells, after 3 and after 2 injections respectively. Moreover, it did strongly decrease the number of immature NKs (CD161+)after 2 injections (Figure 4A-4B).

### Summary

In the present example an immune monitoring (IM) method, based on the FACS detection of 19 surface markers is shown, which can be used in combination, in order to differentiate T-cells, NKs and NKTs and their status as active, inactive, de-activated, resting, immature, mature. Moreover, the present IM can differentiate cytotoxic T-cells from T-reg and T-mem. T-mem are further differentiated as central memory and intermediate memory. The cells are as well characterized as able to respond to DCs stimuli, to produce ILs and INF-gamma. The data can be confirmed by ELISA assays and by co-culture with DCs.

The IM is based on the phenotypical analysis of surface markers by FACS. The analysis is quick, easy and cost-effective. The test only requires 1 mL of peripheral blood from the patient. The blood sample is immediately processed by FACS.

The IM constitutes an optimal tool to determine the effectiveness of experimental treatment for cancer patients, such as immune therapy, based on the inoculation of DCs. The IM is particularly suitable for trials aimed to find out the optimal dose and route administration of experimental medical/pharmaceutical products.

The method can be used in patients before, during and after cancer therapies, in particular in relation to the effectiveness and the improvement of the immune therapy, based on the inoculation of in vitro cultured autologous DCs.

## Claims

1. Method for determining specifically and for discriminating T cells, Natural Killer (NK) cells and Natural Killer T (NKT) cells in a sample comprising the steps of:
a) providing a sample comprising leukocytes, in particular T cells, NK cells and NKT cells, or parts thereof,
b) incubating said sample with at least eight antibodies binding to at least eight different T cell surface markers, NK cell surface markers and NKT cell surface markers, wherein the T cell surface marker is selected from the group consisting of CD3, TCR α/β, TCR γ/δ, CD4, CD8, CD45RO, CD25, CD28, CCR4, CD94, CD38, CD62L and CCR7, the NK cell surface marker is selected from the group consisting of CD56, CD28, CD69, CCR4, CD158a, Nk-p44, CD94, CD38, CD62L, CD8, HLA-DR, CD25 and CD161, and the NKT cell surface marker is selected from the group consisting of CD56, CD3, CD8, CCR4 and CD69, wherein the antibodies used for the incubation bind to at least one T cell surface marker, at least one NK cell surface marker and at least one NKT cell surface marker,
c) detecting the binding of at least one of said antibodies to said cells or parts thereof, and
d) determining T cells, NK cells and/or NKT cells in the sample when at least one of said antibodies binds to said cells or parts thereof and/or determining T cells in the sample when the binding of antibodies directed to CD3, TCR α/β, TCR γ/δ, CD4, CD8, CD45RO, CD25, CD28, CCR4, CD94, CD38, CD62L and CCR7 to said cells or parts thereof and no binding to a NK cell surface marker or NKT cell surface marker is detected, determining NK cells in the sample when the binding of antibodies directed to CD56, CD28, CD69, CCR4, CD158a, Nkp44, CD94, CD38, CD62L, CD8, HLA-DR, CD25 and CD161 to said cells or parts thereof and no binding to a T cell surface marker or NKT cell surface marker is detected and/or determining NKT cells in the sample when the binding of antibodies directed to CD56, CD3, CD8, CCR4 and CD69 to said cells or parts thereof and no binding to a T cell surface marker or NK cell surface marker is detected.

2. Method according to claim 1, **characterised in that** active T cells, NK cells and/or NKT cells present in the sample are determined by detecting the binding of at least one of said antibodies to said cells or parts thereof, wherein active T cells are determined when the binding of antibodies to CD45RO, CD25, CD28, CCR4, CD94, CD38 and/or CD62L to cells in the sample is detected, active NK cells are determined when the binding of antibodies to CD69, CD28, CD158a, Nkp44, CD94, CD38, CD25 and/or CD62L to cells in the sample is detected, and active NKT cells are determined when the binding of antibodies to CD8 and/or CD69 to cells in the sample is detected.

3. Method according to claim 1, **characterised in that** inactive NK cells present in the sample are determined by detecting the binding of at least one of said antibodies to said cells or parts thereof, wherein inactive NK cells are determined when the binding of antibodies to CCR4 to cells in the sample is detected.

4. Method according to claim 1, **characterised in that** resting T cells present in the sample are determined by detecting the binding of at least one of said antibodies to said cells or parts thereof, wherein resting T cells are determined when the binding of CD45RO is simultaneous to the binding to CD4 or CD8 antibodies.

5. Method according to claim 1, **characterised in that** immature NK cells present in the sample is determined by detecting the binding of at least one of said antibodies to said cells or parts thereof, wherein immature NK cells are determined when the binding of antibodies to CD161 and/or HLA-DR to cells in the sample is detected.

6. Method according to claim 1, 2 or 4, **characterised in that** the T cells present in the sample are differentiated in cytotoxic T cells, regulatory T cells and memory T cells, wherein cytotoxic T cells are determined when the binding of antibodies to CD8 and/or TCRα/β to cells in the sample is detected, regulatory T cells are determined when the binding of antibodies to CD4 and/or CD25 to cells in the sample is detected and memory T cells are determined when the binding of antibodies to CCR7 to cells in the sample is detected.

7. Method according to any one of claims 1 to 6, **characterized in that** the sample is blood, lymphonodal fluid, tumor lysate, leukaphereses and elutriation.

8. Method according to any one of claims 1 to 7, **characterized in that** the antibodies are labelled, preferably fluorescently labelled.

9. Method according to any one of claims 1 to 8, **characterized in that** the label is selected from the group consisting of fluorescein isothiocyanate (FITC), phycoerythrin (PE), allophycocyanin (APC), PE-cyanin-5 (PC5), PE-cyanin-7 (PC7), PE-cyanin-5.5, PE-Texas Red (ECD), rhodamine, PerCP and Alexa dyes.

10. Method according to any one of claims 1 to 9, **characterized in that** the detection of binding of the antibodies to the cells is performed by flow cytometry.

11. Method according to any one of claims 1 to 10, **characterized in that** the cells detected in the sample are quantified.

12. Kit for determining specifically and for discriminating T cells, Natural Killer (NK) cells and Natural Killer T (NKT) cells in a sample comprising antibodies directed to CD3, TCR α/β, TCR γ/δ, CD4, CD8, CD45RO, CD25, CD28, CCR4, CD94, CD38, CD62L, CCR7, CD56, CD69, CD158a, Nkp44, CD38, HLA-DR and CD161, wherein the antibodies are fluorescently labelled.

13. Kit according to claim 12, **characterized in that** the label is selected from the group consisting of fluorescein isothiocyanate (FITC), phycoerythrin (PE), allophycocyanin (APC), PE-cyanin-5 (PC5), PE-cyanin-7 (PC7), PE-cyanin-5.5, PE-Texas Red (ECD), rhodamine, PerCP and Alexa dyes.

14. Use of a kit according to claim 12 or 13 for determining specifically and for discriminating T cells, Natural Killer (NK) cells and Natural Killer T (NKT) cells in a sample.
